# EUROPÄISCHE PATENTANMELDUNG

(11) **EP 0 591 893 A1**
(43) Veröffentlichungstag der Anmeldung: **13.04.1994**
(21) Anmeldenummer: 93116002.2
(22) Anmeldetag: 04.10.1993
(51) Int. Cl.: G21K 1/00, A61N 1/16

(54) **Verfahren zur Aufladung von Wasser und Räumen mit kosmischer Energie**

(30) Priorität: 06.10.1992 DE 4233515
(71) Anmelder: Kehlbeck, Heinrich, D-27318 Hoya (DE)
(72) Erfinder: Kehlbeck, Heinrich, D-27318 Hoya (DE)
(74) Vertreter: Minderop, Ralph H., Dr. rer. nat.

(57) **Zusammenfassung**

Es wird ein Verfahren zur Aufladung von Wasser und Räumen mit kosmischer Energie beschrieben, in dem ein einseitig offenes Kunststoff- oder Edelstahlröhrchen eines Durchmessers von bis zu 10 cm oder eine eckige oder runde Platte entsprechender Größe zum Auffangen und Weiterleiten der Energie senkrecht mit der offenen Seite nach oben aufgestellt wird.

## Beschreibung

Die Erfindung betrifft ein Verfahren und die Verwendung geeigneter Vorrichtungen zur Behandlung von elektropositiv verunreinigten Räumen, Gewässern, Wasser, Arznei- und Lebensmitteln.

Ohne das sich durch verschiedene Eigenschaften von anderen Molekülen unterscheidende Wasser ist Leben undenkbar. Einige solcher Eigenschaften sind beispielsweise die Dichteanomalie und die vergleichsweise außerordentlich hohe Dielektrizitätkonstante. Die Polarisierung der O-H-Bindung geht einher mit der Fähigkeit zur Ausbildung von Wasserstoffbrückenbindungen und damit auch zur Bildung einer Struktur im flüssigen Wasser. In diesem herrscht ein Ordnungszustand, der noch nicht bis in alle Einzelheiten geklärt ist. Aus quantenmechanischen Berechnungen und Ergebnissen von Röntgen- und Neutronenbeugungen, IR- und Raman-Spektroskopie wurde u. a. das Cluster-Modell abgeleitet. Man nimmt an, daß durch Wasserstoffbrückenbindungen zusammengehaltene Aggregate eine dem Eis analoge Struktur bilden.

Chemische Systeme wie Moleküle oder chemische Bindungen besitzen jeweils eine bestimmte potentielle Energie. Dies gilt auch für Wasser, denn es bekannt, daß sich zwischen laminaren Schichten im Wasser oder in Gewässern eine elektrische Spannung als Ergebnis der unterschiedlichen Energiegehalte zweier oder mehrerer Schichten aufbaut. Ein hoher Energiegehalt wird insbesondere dem Quellwasser zugeschrieben, welches sowohl als Lebensmittel und als Heilmittel verwendet wird. Hochwertiges Gebirgsquellwasser unterscheidet sich von atmosphärischem Wasser oder Oberflächenwasser durch seine chemische Zusammensetzung, Temperatur, Dichte und auch hinsichtlich des elektrischen Potentials. Unter atmosphärischem Wasser werden alle Arten von Wasser, die vom Menschen aufbereitet worden sind, einschließlich destilliertem Wasser sowie jenen Wasserarten, die einem Übermaß an starker Licht-, Druck- und Temperatureinwirkung ausgesetzt waren, verstanden. Das an der Quelle heraustretende Gebirgswasser enthält gelöste Mineralstoffe und einen relativ hohen Gehalt an Gasen - hauptsächlich Kohlendioxid - in freier und gebundener Form. Dieses Wasser ist sehr kühl und von hoher Dichte, es ist moussierend bzw. sprudelnd. Wasser mit einem hohen Gehalt an Spurenelementen und Kohlendioxid weist bei kühlen Temperaturen wesentlich mehr Energie auf als Oberflächen- und atmosphärisches Wasser, das einen begrenzten Gehalt an Mineralsalzen und einen höheren Gehalt an freien Sauerstoffradikalen hat.

Durch die Verwendung von galvanisierten Stahlrohren verliert das Wasser nicht nur seine elektrische Ladung, sondern auch die Mineralsalze, die eine Regenerierung biologischer Systeme bewirken sollten.

Es ist eine bekannte Tatsache, daß der durch Druck bewirkte Transport des Wassers durch herkömmliche Pumpen und gerade Röhren einen Prozess der molekularen Desintegration auslöst. Bei dieser zentrifugal induzierten Reaktion wird die Lebensenergie des Wassers entladen. Es konnte nachgewiesen werden, daß Wasser, welches diesem Druck und Temperaturanstieg ausgesetzt wurde, sich innerhalb von 24 Stunden zu einer Brutstätte von Bakterien und Mikroorganismen entwickelte, was Desinfektionsverfahren erforderlich machte.

Es ist auch bekannt, daß manche chemische Verbindungen, sogenannte chaotrope Stoffe, in Wasser gelöst, dessen Ordnungszustand herabsetzen, indem sie die Wasserstoffbrücken zerstören (Römpps Chemie-Lexikon, 8. Aufl., Bd. 6, S.4584 re. Sp.).

Diese Art der Verschmutzung von Wasser oder Gewässern wird in der Geobiologie als sogenannte elektropositive Verschmutzung bezeichnet, die sich sowohl qualitativ als auch quantitativ nachweisen läßt. In der chinesischen Volksmedizin wird dieser Zustand auch als "yin" bezeichnet.
Eine sogenannte elektropositive Verschmutzung tritt aber nicht nur im Wasser, sondern in Räumen aller Art und auch bei dort gelagerten Lebens- und Arzneimitteln auf. Sie kann dort durch Strom führende Strahlungsquellen wie Computer, Telefone, Kühlschränke sowie deren Leitungen, d.h. Elektrizitätsverbraucher und Sendeeinrichtungen aller Art verursacht sein.

In Arbeitsräumen mit hochtechnisierten Geräten oder mit Wechselstrom betriebenden Maschinen, wo die Böden mit Kunststoff, Vliesen oder Beton isoliert sind, besteht eine elektropositive Aufladung, wovon auch die darin arbeitenden Personen betroffen sind. Sie befinden sich im sogenannten Yin-Zustand. Dieser Zustand wird veranlaßt durch Wechselstrom, für den Organismus gefährliche Stoffe und Störzonen aller Art.

Der hier verwendete Begriff "elektropositiv" ist nicht den in der Chemie verwendeten Begriffen der Elektropositivität oder Elektronegativität gleichzusetzen. Elektropositiv im Sinne der vorliegenden Erfindung äußert sich vielmehr in einer kreisförmigen Bewegung einer Schuhmacher Sonde gemäß der europäischen Patentanmeldung 90 111 271.4 entgegen dem Uhrzeigersinn.

Eine Reihe von Verfahren wurden zur energetischen Aufbesserung des Wassers bereits vorgeschlagen, wobei das alleinige Anreichern mit Mineralstoffen und Schütteln des angereicherten Wassers nicht ausreicht, dieses energetisch aufzuladen.

So wird mit einem sogenannten Quellwasser-Generator, der auch in der Bundesrepublik Deutschland erhältlich ist, versucht, die natürlichen geosphärischen Prozesse, die Quellwasser entstehen lassen, nachzuvollziehen. Dem Wasser werden zunächst Spurenelemente und Kohlendioxid zugeführt und diese Mischung dann in besagter Einrichtung einer zentripedalen, wirbel-bzw. strudelartigen Bewegung bei vollkommener Dunkelheit und einer Temperatur von 4°C ausgesetzt.

Aus Raum & Zeit 49/91, S. 3 - 9 ist ein sogenanntes Levitationsverfahren bekannt, durch das Wasser strukturell so verändert werden soll, daß es aus seinem gravitativ geprägten, stabilen energetischen Gleichgewichtszustand in einen auf einem höheren Energieniveau liegenden metastabilen Gleichgewichtszustand angehoben wird. Dies geschieht durch starke Beschleunigung des Wassers in einer Vorrichtung zur Levitation, in der die Wasserbewegung zwei ineinandergeschachtelte Spiralen beschreibt.

Beim Einlauf in den Innenbehälter wird der Drehsinn der Strömung verändert. Man geht davon aus, daß die in das Wasser eingetragene Schwingungsenergie zu einer höheren Anzahl an offenen Wasserstoffbrückenbindungen führt, wodurch der Informationstransport im Wasser selbst erhöht werden soll. Diese Wirkung manifestiere sich dadurch, daß sich der kolloidal gelöste Anteil von mineralischen Dispersionen erheblich steigern ließe und Emulsionen mit levitiertem Wasser stabiler seien, so daß der Anteil an Emulgator reduziert werden könne. Auch diese Vorrichtung wird auf dem deutschen Markt angeboten.
Diese Verfahren eignen sich jedoch nur zur Revitalisierung von Wasser.

Im Jahre 1912 wurde von dem österreichischen Physiker Victor F. Heess die kosmische Strahlung entdeckt und deren Eigenschaften in den folgenden Jahrzehnten allmählich erforscht. Den Hauptbestandteil dieser Strahlung bilden Atomkerne ohne Elektronenhülle, die sich nahezu mit der Geschwindigkeit des Lichts bewegen. Der Energiegehalt dieser schnellen Atomkerne ist so groß, daß die Leistung, die in unserer Milchstraße als kosmische Strahlung freigesetzt wird, weitaus größer ist als die in Form von Röntgenstrahlung oder Radiowellen. Die energiereichsten Teilchen sind oberhalb von 10²⁰ Elektronenvolt beobachtet worden. Das untere Ende des Spektrums der kosmischen Strahlen ist verhältnismäßig unscharf definiert, jedes Photon (Quant der elektromagnetischen Strahlung) oder Teilchen mit einer Energie von mehr als 10⁸ Elektronenvolt, das aus dem Weltraum eintrifft, wird als kosmischer Strahl bezeichnet (Spektrum der Wissenschaften, Januar 1986, S. 52).

Es ist bekannt und leuchet ein, daß kosmische Energie das Leben auf unserer Erde beeinflußt, wobei im Gegensatz zu den durch Fernseh-, Funk- und Mikrowellen verursachten Wellen und den elektrischen Feldern aller möglichen elektrischen Geräte und Leitungsnetzen ein positiver Einfluß auf die Gesundheit angenommen wird. Voraussetzung für eine erfolgreiche Anreicherung mit kosmischer Energie ist zunächst die Neutralisierung der elektropositiven Verschmutzungen.

Der Erfindung liegt somit die Aufgabe zugrunde, sogenannte kosmische Energie zur energetischen Verbesserung von elektropositiv verschmutztem Wasser, Gewässern und Räumen und darin vorhandenen Lebens- und Arzneimitteln nutzbar zu machen.

Diese Aufgabe wird gelöst durch die Verwendung von einseitig offenen Kunststoff- oder Edelstahlgefäßen mit einem Durchmesser von etwa 0,3 cm und mehr oder Platten entsprechender Größe zum Auffangen und Weiterleiten von Energie.

Es wird angenommen, daß bei Anwendung der erfindungsgemäß einzusetzenden Vorrichtung in einem Gebäude ein neutraler Schwingungszustand oder neutraler energetischer Zustand erreicht wird, so daß eine Aktivierung mit Photonen-Energie möglich ist. Bei verschmutztem oder mit Chemie belastetem Wasser verhält es sich ähnlich, denn elektropositiv verschmutztes Wasser nimmt keine Photonen auf.

Diese Gefäße können die Form eines einseitig verschlossenen Röhrchens oder einer Dose besitzen.

Die Höhe der erfindungsgemäß eingesetzten Gefäße ist unkritisch, sie beträgt beispielsweise 5 bis 10 cm, kann aber auch wesentlich größer sein, die Wandstärke kann 2 mm oder mehr betragen. Beispielsweise ist eine Bodenstärke von 2 mm oder mehr denkbar.

Der Durchmesser kann beispielsweise 20 cm oder mehr betragen. Geeignet sind auch solche Ausführungsformen, deren Durchmesser der Bodenfläche nicht der Öffnung entspricht. Es sind Öffnungen mit Durchmessern bis 20 cm und mehr denkbar. Die Gefäße können ein Volumen von bis zu 100 1 aufweisen und gegebenenfalls, was die Seite mit der Öffnung anbetrifft, mit einem zur zentralen Öffnung hin geringer werdenden Abstand zur Bodenplatte ausgebildet sein. Die Seitenwände können über die die Öffnung aufweisende Fläche hinausgezogen sein. Es können Einrichtungen zur Weiterleitung der Energie über Kabel vorhanden sein.

Die Kunststoffröhrchen bestehen in geeigneter Weise aus dickwandigem Isoliermaterial. Als besonders geeignet haben sich erwiesen Polyvinylchlorid, Polyethylene hoher und niederer Dichte, Polyvinylidenchlorid, Poly(meth)acrylat, Polyacrylnitril, Polyvinylalkohol, Polyvinylacetat, Polystyrol, Tetrafluorethylen-Hexafluorpropylen. Diese Kunststoffe können behandelt, beispielsweise einer Härtung oder Färbung unterzogen sein.

Die erfindungsgemäß einsetzbaren Platten bestehen aus den gleichen Materialien, die Stärke kann 2 mm und mehr, beispielsweise 1 cm betragen.

Die erfindungsgemäß herbeigeführte Wirkung läßt sich mit Röhrchen aus Holz, Keramik, Glas, Stein oder Eisen nicht herbeiführen.

Eine erhebliche Verstärkung der Energieaufnahme und Abstrahlung ergibt sich durch eine gebündelte Aufstellung mehrerer Edelstahlgefäße. Um ein zentral angeordnetes Edelstahlgefäß herum sind dann weitere Edelstahlgefäße angeordnet. Bei gebündelten Kunststoffgefäßen hingegen bricht die Abstrahlung zusamman, wenn nicht die Bündelung durch einen Metalldraht, beispielsweise Silberdraht oder mit einer Metallplatte erfolgt.

Die Verstärkung der Platte oder der Bodenplatte der Röhrchen oder runden Gefäße führt auch zur Erhöhung der Abstrahlung.

Diese Wirkung tritt dann nicht ein, wenn das Röhrchen mit der offenen Seite in der der Wasseroberfläche abgewandten Seite ausgerichtet wird. Die Wirkung ist unverändert stark, wenn die erfindungsgemäß eingesetzen Gefäße etwa zwei Meter unter der Wasseroberfläche oder entsprechend tief im Erdreich aufgestellt werden.

Zur Durchführung des erfindungsgemäßen Verfahrens werden die Röhrchen senkrecht, mit dem offenen Ende nach oben, aufgestellt. Dies reicht aus, um einen starken Energieausstoß, der sich nach oben hin verbreitert, zu bewirken. Dieser Energieausstoß ist so stark, daß er sich durch Betondecken fortpflanzt. Befestigt man in den Röhrchen Elektroden und verbindet diese mit einem Kabel, kann die aufgefangene Energie über mehrere tausend Meter beliebig abgelenkt werden. Werden diese Rohre in ein Wasserbecken, in Teiche, Seen, Flüsse oder in die Wassertanks der öffentlichen Wasserversorgung gestellt, erfolgt dadurch eine Aufladung des Wassers mit Energie, wobei diese Energie gegebenenfalls über einen langen Zeitraum von mehreren Jahren darin erhalten bleibt.

So können die erfindungsgemäß eingesetzten Vorrichtungen die Form von Trinkgefäßen oder Pflanzengefäßen aufweisen, um eine bessere energetische Versorgung des jeweiligen Inhalts, Getränk oder Pflanzen, zu ermöglichen oder zur Behandlung von Gießwasser die Form einer Gießkanne oder Eimers besitzen. Die erfindungsgemäß eingesetzen Vorrichtungen, eckiger oder runder Form, können in Wohn- und Arbeitsbereichen, Lagerhallen für alle Arten von Lebens- oder Arzneimitteln oder Fertigungsräumen wie Bäckereien, Käsereien, Brauereien zur Verbesserung des energetischen Zustands der dort gelagerten oder hergestellten Produkte ausgelegt werden.

Wird ein Waschbecken oder eine Badewanne mit elektropositiv verunreinigtem Wasser aufgefüllt und unter der Wasseroberfläche ein kleines, erfindungsgemäß zu verwendendes Röhrchen mit der Öffnung nach oben aufgestellt, ist das Wasser in wenigen Sekunden bis Minuten aufgeladen und geschwächte Personen spüren nach Kontakt mit diesem Wasser eine günstige Veränderung.

Ein weiterer Nachweis der erfindungsgemäßen Wirkung erfolgt mit einem sogenannten Sensor, mit dem die elektropositive Energie beispielsweise über einem Telefon durch Schwingen eines Teils des Sensors in kreisförmigen Bahnen entgegen dem Uhrzeigersinn dargestellt wird. Bei Aufstellung einer erfindungsgemäß eingesetzten Vorrichtung im Raum, läßt das Schwingen der Sonde nach bis zu deren Stillstand, was als Zurückdrängen der elektropositiven Energie gedeutet wird.

Mit der Schuhmacher Sensoreinrichtung läßt sich ein qualitativer und halbquantitativer Nachweis der Energie durchführen.

Die Anreicherung des Wassers mit Energie läßt sich auch qualitativ messen. Dazu steht das REDEM-System zur Verfügung, welches in der DE-A-37 09 665 beschrieben ist. In diesem Verfahren wird die zu analysierende Probe als Dieelektrikum in einen Kondensator eingebracht, der an einen Schwingkreis gekoppelt ist. Je nach Frequenz des verwendeten Schwingkreises und der Natur der Probe wird der Schwingkreis mehr oder weniger stark gedämpft, verglichen mit dem Schwingungsverhalten der Vergleichsprobe. Das Schwingkreissystem muß bis auf die Änderung des Meßkondensators durch die eingebrachte Probe thermisch und zeitlich frequenz- und amplitudenstabil sein.

Die wissenschaftliche Beweisführung wird vordringlich mit einem Meßgerät für Bio- und Chemiluminiszenz vorgenommen, welches einen hochempfindlichen Photomultiplier enthält.

## Patentansprüche

1. Verfahren zur Anreicherung von Wasser und von Räumen oder darin gelagerten Lebens- und Arzneimitteln mit kosmischer Energie, **dadurch gekennzeichnet**, daß ein einseitig offenes Kunststoff- oder Edelstahlgefäß eines Durchmessers von 0,3 cm und mehr oder eine eckige oder runde Platte entsprechender Größe zum Auffangen und Weiterleiten der Energie senkrecht mit der offenen Seite nach oben aufgestellt wird.

2. Verfahren nach Anspruch 1, **dadurch gekennzeichnet,** daß die Gefäße an ihrer offenen Seite eine Öffnung eines Durchmessers von 0,3 cm und mehr aufweisen.

3. Verfahren nach Anspruch 1 oder 2, **dadurch gekennzeichnet,** daß mehrere Edelstahlgefäße gebündelt eingesetzt werden.

4. Verfahren nach Anspruch 1 oder 2, **dadurch** gekennzeichnet, daß mehrere Kunststoffgefäße gebündelt eingesetzt werden, wobei die Bündelung mit einem Metalldraht erfolgt oder die gebündelt aufgestellten Gefäße auf einer Metallplatte angeordnet sind.

5. Verfahren nach Anspruch 1 bis 4, **dadurch gekennzeichnet**, daß die Platten oder Röhrchenböden eine Stärke von 0,2 bis zu 1 cm oder mehr aufweisen.

6. Verwendung einseitig offener Kunststoff- oder Edelstahlgefäße eines Durchmessers von 0,3 cm oder mehr oder einer eckigen oder runden Platte entsprechender Größe zur Anreicherung von Wasser und von Räumen oder darin gelagerten Lebens- und Arzneimitteln mit kosmischer Energie.
